# EUROPEAN PATENT SPECIFICATION

(11) **EP 2 236 524 B1**
(45) Date of publication and mention of the grant of the patent: **16.09.2015**
(21) Application number: 09004566.7
(22) Date of filing: 30.03.2009
(51) Int. Cl.: C08F 8/30, A61Q 3/02, A61K 8/65, A61K 8/84, C03C 17/32

(54) **Adhesion promoter based on a functionalized macromolecule comprising photoreactive groups**
Haftpromoter mit Polymer das eine fotoreaktive funktionelle Gruppe beinhaltet
Promoteur d'adhésion à base d'une macromolécule functionalisée comprenant des groupes photoréactifs

(43) Date of publication of application: 06.10.2010
(73) Proprietor: SuSoS AG, 8600 Dübendorf (CH)
(72) Inventor: Zürcher, Stefan, 8050 Zürich (CH); Tosatti, Samuele, 8050 Zürich (CH); Dorcier, Antoine, 5022 Rombach (CH); Fusco,Stefan, 8057 Zürich (CH); Lopez, Ignacio, 8004 Zürich (CH)
(74) Representative: Schaad, Balass, Menzl & Partner AG

(56) References cited:
- WO-A1-2006/063181
- WO-A2-2004/076511
- US-A- 6 022 597
- US-A1- 2007 154 888

## Description

The present invention relates to a functionalized macromolecule, a composition comprising said functionalized macromolecule, and to its use as an adhesion promoter.

It is known to use an adhesion promoter to improve the adhesion between two different materials, in particular between a substrate and a coating. Typically used as adhesion promoters are compounds comprising reactive groups like silanes, titanates, chromium complexes or unsaturated carboxylic acids. There are various applications for such adhesion promoters, for example in the automobile, packaging or construction industry, but also for dental prostheses.

Permanent coating of hydrophobic polymers, such as polytetrafluoroethylene (PTFE, Teflon®) or polypropylene (PP), is often difficult, due to only weak interactions - typically based on van der Waals interactions - between substrate and coating. By activating the substrate using physical methods, such as plasma, corona or chemical etching methods, it is possible to momentarily overcome these adhesion problems. However, the thus activated substrates will loose these properties again within a relatively short period of time, as the thus activated surface is not stable and will rearrange back to the original state, prior to the activation treatment. In addition, these very harsh methods are sometimes not feasible for sensitivity reasons or very costly.

Another field of application for adhesion promoters is fingernail enhancements. For aesthetic and practical reasons, natural fingernails are covered with a nail filling gel or with artificial fingernails. This process is usually performed in specialized nail studios. During a first visit, a customer's fingernails are coated with the gel, which is typically a UV curable polymer. After this first, somewhat more complex visit, the customer has to return to the nail studio on a monthly basis to fill up the gaps created due to nail growth.

The main problems of these nail filling gels are related to premature detachment of the polymer coating, typically caused by mechanical stress, formation of air bubbles between gel and nail or poor adhesion due to the presence of an intermediate layer of fatty organic compounds.

In order to improve their mechanical properties,brittle and soft fingernails are covered with a so-called fingernail hardener. The application of such a nail hardener is usually similar to the application of nail polish: The fingernail is painted with the nail hardener composition, and upon evaporation of the solvent contained therein, the nail hardener is cured. UV curable fingernail hardeners are also known.

It is a problem of the present invention to provide the means for obtaining a permanent coating on "difficult" substrates, such as hydrophobic surfaces or fingernails, for instance.

It is another problem of the present invention to provide an effective adhesion promoter, the application of which is uncomplicated and does not require extensive training of the operator.

US 6 022 597 discloses a method for covalently modifying surfaces of various substrates. The surfaces are exposed to a reagent, having molecules each comprising a nitrogenic group and a functionalizing group, in the presence of energized charged particles such as electrons and ions, photons, or heat, which transform the nitrogenic reagent to a nitrene intermediate. The nitrene covalently reacts with any of various chemical groups present on the substrate surface. The functionalizing groups can then participate in downstream chemistry whereby any of a large variety of functional groups can be covalently bonded to the surface.

WO 2006/063181 relates to medical articles that include more a polymeric matrix including more than one biocompatible agent, wherein each biocompatible agent is provided at a distinct portion of the medical article surface.

WO 2004/076511 discloses a hydrogel layer, which is applied to a substrate, using a polymeric or copolymeric precursor that includes monomer subunits that have a photocrosslinkable functionality and monomer subunits that have a chemically selective functionality for binding a biomolecular analyte, such as a protein.

US 2007/154888 relates to a method for the covalent immobilization of probe biomolecules on organic surfaces, by means of photoreactive cross-linking agents, which are used to covalently immobilize the probe biomolecules to soluble polymers or copolymers that are then covalently immobilized on an organic surface.

The problem is solved by the functionalized macromolecule according to claim 1, the composition according to claim 4, and the uses according to claims 6 to 10. Further preferred embodiments are subject of the dependent claims.

The functionalized macromolecule according to the present invention comprises a polymeric core and at least two functionalized side chains. The polymeric core comprises a synthetic polymer selected from the group consisting of polyallylamine (PAAm), polyethyleneimine (PEI). The term "keratin", throughout this application, does not only include pure natural and/or synthetic keratin, but also protein mixture obtained by extracting keratin containing natural materials, such as hair, feathers or claws, for instance. The at least two functionalized side chains are linked to the polymeric core by a linker group A selected from the group consisting of a secondary or tertiary amine, an ether, a thioether, a carboxylic acid ester, an amide and a thioester. The at least two functionalized side chains comprise a photoreactive group, wherein said photoreactive group is

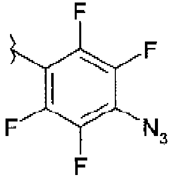

One example of the functionalized macromolecule according to the present invention is polyallylamine-grafted-perfluorophenylazide (PAAm-g-PFPA), which is shown below:

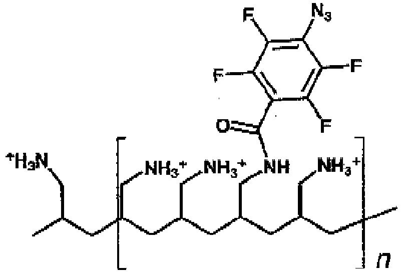

In the above case, the polymeric core is polyallylamine, the linker group A is an amide group and the photoreactive group is para-perfluorophenylazide. On average, about every 4^{th} repeating unit of the polyallylamine core

The polymeric core of the functionalized macromolecule according to the present invention allows for a fine-tuning of the physical properties of the macromolecule with respect to solubility, melting and boiling point, hydrophilicity or hydrophobicity, and positive or negative charge by choice of the polymer. Thereby, non-covalent interactions between the macromolecule and a substrate to be coated can be adjusted according to specific requirements.

Preferably, the synthetic polymer of the polymeric core has a molecular weight of about 1 kDa to 5 000 kDa, more preferably of about 10 kDa to 2 500 kDa. For example, the polymeric core may comprise polyallylamine of about 14 kDa or a bovine serum albumin of about 66 kDa.

Prior to an activation, the functionalized macromolecule of the present invention is basically inert and can be applied to and removed from a substrate very easily, as there are only weak interactions - such as van der Waals, hydrophobic or electrostatic interactions or hydrogen bonding - between the macromolecule and the substrate.

Thanks to the two or more functionalized side chains, the reactivity of the macromolecule of the present invention can be controlled, in particular with respect to the initiation of a reaction with a substrate and/or an overlayer and to the strength of the bonding formed between the functionalized macromolecule and the substrate and/or overlayer. By means of the photoreactive group, the formation of covalent bonds can be initiated by UV radiation cr heat, leading to very reactive intermediates, which undergo reaction with the substrate and/or overlayer. It is believed that upon activation, the photoreactive group undergoes a non-specific insertion reaction into a nearby C-X bond, such as a C-H, C-N C-O, C-C or C-F bond, for instance. The insertion reaction may take place between an activated photoreactive group and a C-X bond within the substrate, the overlayer, if present, or a macromolecule of the present invention. The conversion rate for the covalent bond forming reaction is much higher than the rate of an unproductive radical formation or hydrogen abstraction. Depending on the number of functionalized side chains and the photoreactive group chosen, the strength of bonding between the macromolecule and the substrate and/or overlayer can be designed to be more or less strong, as desired/required. The polymeric core of the functionalized macromolecule according to the present invention is polyallylamine or polyethyleneimine. Polyallylamine and polyethyleneimine allow for a simple and straightforward coupling to the functionalized side chains. In addition, these polymers are easily available, inexpensive and very well water soluble, as they comprise a large number of positively charged groups at neutral pH.

In a preferred embodiment, the linker group A connecting the polymeric core with a functionalized side chain is an amide. This functional group can be introduced very easily, for example by standard coupling of an amine with an acid chloride or a reactive ester, such as *N-*hydroxysuccinimide (NHS), pentafluorophenol or *in situ* EDC/NHS standard peptide chemistry. In addition, the amide linkage is relatively stable under typical environmental conditions.

The photoreactive group is perfluorophenylazide (PFPA), i.e. a substituent of the formula

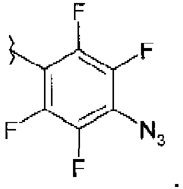

This photoreactive group allows for a fast and very efficient curing of the adhesion promoter by UV irradiation, preferably with a wavelength of less than 400 nm, or heat, preferably at a temperature of at least 120 °C. Upon activation, the azide group is converted to a nitrene, which undergoes a very fast, non-specific insertion reaction into a nearby C-X bond of the substrate, a functionalized macromolecule or the overlayer, if present.

In a preferred embodiment, the polymeric core of the functionalized macromolecule comprises on average a functionalized side chain on at least every 24^{th}, preferably on at least every 12^{th}, more preferably on at least every 4^{th} repeating unit or amino acid unit, respectively. In the case of a polymeric core comprising of a protein, the functionalized side chain is typically attached to the side chain of the amino acid unit. The distribution of the side chains is usually statistical. Thanks to the multiple side chains, it is possible to form a permanent covalent bonding between the adhesion promoter and both the substrate and the functional overlayer, if present. The more functionalized side chains the macromolecule comprises, the better is the crosslinking between substrate, adhesion promoter and potential additional layers.

In a further aspect, the present invention relates to a composition comprising the functionalized macromolecule of the present invention and a liquid. The liquid is preferably water, a water mixable solvent or a mixture thereof. More preferably, the liquid is selected from the group consisting of water, acetone, ethanol, methanol, 2-propanol, *N,N*-dimethylformamide, dimethylsulfoxide, and mixtures thereof. Optionally, the water is buffered with one of the standard buffer systems known to a person skilled in the art, for instance with HEPES (2-(4-(2-hydroxyethyl)-1-piperazinyl)-ethanesulfonic acid).

The composition of the present invention is relatively stable under normal conditions and can be easily handled and stored. For use, the composition is directly applied to a substrate. Upon the application, the functionalized macromolecule binds to the substrate surface by means of weak interactions, such as van der Waals, hydrophobic or electrostatic interactions or hydrogen bonding. The liquid, on the other hand, is fully or partially evaporated. Upon UV irradiation and/or heating, the functionalized macromolecule is activated and undergoes covalent bond forming reactions with the substrate and, if present, also with an additional coating layer. Thereby, a very strong and long lasting connection between the substrate, the functionalized macromolecule and, if present, an additional layer is formed.

Apart from coating metal and metal oxide surfaces, such as glass, silicon, silicon dioxide, iron, iron oxide, titanium or titanium dioxide, for instance, the composition of the present invention is in particular also suited for coating "difficult" substrates, such as hydrophobic surfaces or fingernails, for instance.

In a preferred embodiment, the composition of the present invention further comprises an additive selected from the group consisting of polyethylene glycol, polyvinylpyrrolidone, dextran, fatty acids, keratin, collagen, and mixtures thereof. These additives may serve as filler materials or as additional active agents, improving the properties of the composition. These additives may also convey additional, advantageous properties to the composition, such as hardness, flexibility, low friction, opaqueness, anti-fogging, protein or cell resistance properties

A further aspect of the present invention relates to the use of the above composition as an adhesion promoter.

The composition is particularly well suited as an adhesion promoter, as it is relatively stable under normal conditions and can be easily handled and stored. Furthermore, the composition of the present invention is nontoxic. In addition, the application of the composition to a substrate is very straight forward: The composition can be sprayed onto the substrate or the substrate can be immersed in the composition, for instance. Upon the application, the functionalized macromolecule binds to the substrate surface by means of weak interactions, such as van der Waals, hydrophobic or electrostatic interactions or hydrogen bonding. Thereby, a temporary connection between the functionalized macromolecule and the substrate is formed, allowing for further processing of the coated substrate, for instance with an additional overlayer.

The liquid comprised in the composition, on the other hand, is fully or partially evaporated. Upon UV irradiation and/or heating of the coated substrate, the functionalized macromolecule is activated and undergoes covalent bond forming reactions with the substrate and, if present, also with an additional coating layer. Thereby, a very strong and long lasting connection between the substrate, the functionalized macromolecule and, if present, the additional coating layer is formed.

In a preferred embodiment, the composition of the present invention is used as an adhesion promoter for nail enhancements, such as cosmetic nail extensions, artificial fingernails, and/or nail modeling and repair systems. The composition is particularly well suited for these applications, as it is very easily applied to the fingernails, is able to infiltrate and fill up all the indentations usually present in the surface of natural fingernails, and guarantees a long lasting fixation of the nail enhancement product on the fingernail. The composition is simply spread onto the fingernail and then air dried to remove the liquid component. After the application of the artificial fingernail or nail filling gel, UV irradiation causes the formation of covalent bonds between nail, adhesion promoter and nail enhancement. In addition, as many nail filling gels are based on a UV curable polymer, it is possible to cure the adhesion promoter and the nail filling gel at the same time in a single procedural step. In order to fine-tune the mechanical properties of the adhesion promoter, for instance with regard to elasticity, additives, such as PEG, keratin or protein mixtures obtained by extracting natural keratin containing materials, such as hair, feathers, claws, etc., may be added.

In another preferred embodiment, the composition of the present invention is used as a fingernail hardener. The application is again very simple, as the composition is spread onto the fingernail and then air dried to remove the liquid component. The nail hardener of the present invention renders the fingernails harder, more flexible and less prone to breaking, and at the same time levels the surface of the nail. The mechanical properties of the nail hardener can be tailored by the addition of functional additives, such as hydrophilic polymers (e.g. PEG or PVP) or protein mixtures obtained by extracting natural keratin containing materials, which further enhance the performance of the nail hardener.

In another preferred embodiment, the composition of the present invention is used as an adhesion promoter for hydrophobic polymer substrates, such as polytetrafluoroethylene (PTFE, Teflon®) or polypropylene (PP). Again, the application of the adhesion promoter is very straightforward, as the substrate can simply be immersed in the composition, for instance. The composition of the present invention is adsorbed on the hydrophobic surface of the substrate thanks to van der Waals interactions and can be used to bind a coating, for instance a polymer, to the substrate. An overlayer can be applied by dipping, spraying, spincoating, brushing or printing, for instance. Again, the formation of covalent bonds is initiated with UV irradiation or heat. In addition, as the substrate is also a polymer, the adhesion promoter is able to form a network-like, stable structure with the substrate.

In a preferred embodiment, the composition of the present invention is used for permanently binding a polymer coating to a substrate. Depending on the polymer coating, it is possible to vary the properties of the coated substrate surface. For instance, it is possible to render a hydrophobic Teflon® surface permanently hydrophilic by applying the composition and an overlayer comprising a water soluble polymer, such as polyethylene glycol (PEG), polyvinyl alcohol (PVA), polyvinylpyrrolidone (PVP) or dextran, or to render the surface of a hydrophilic polymer hydrophobic, by applying the composition and an overlayer comprising a comprising polymer, such as polyvinylidene fluoride (PVDF), polydimethylsiloxane (PDMS) or a poly-α-olefin (PAO). In addition, using said polymers as overlayers, it is possible to add a variety of different functional properties to the material, such as anti-fog anti-bacterial, non-fouling, non-adhesive or lubricating properties. In this manner, it is, for example, possible to provide substrates like glass, polycarbonate or mirrors with a water soluble hydrophilic polymer as an overlayer, which conveys anti-fog properties to the substrate surface, if the polymer is thick enough, i.e. at least 10 nm, preferably at least 20 nm thick. Thus, in a preferred embodiment, the composition of the present invention is used as an adhesion promoter for an anti-fog coating.

Preferably, the polymer used for the overlayer has a molecular weight of about 1 kDa to about 5 000 kDa, more preferably of about 10 kDa to 2 500 kDa. For example, the polymer of the overlayer may comprises a hydrophilic polymer, such as a polyethylene glycol of about 35 kDa or about 1 000 kDa, a polyvinylpyrrolidone of about 1 300 kDa, a dextran of about 2 000 kDa, a, or a mixture of a polyvinylpyrrolidone of about 1 300 kDa and a polyallylamine grafted with perfluorophenylazide of about 19 kDa.

In another expedient application, the composition of the present invention is used for improving the durability of a printing on a hydrophobic surface. In this case, the composition is applied to the surface to be printed, before the actual printing step, and afterwards UV cured. The printing can be done using conventional methods, such as pad printing, for instance.

In a further aspect, the present invention also relates to a method for applying a functional coating to a substrate surface. The method comprises the steps of:
(a) treating the substrate surface with an adhesion promoter comprising the functionalized macromolecule of the present invention to form a film of the adhesion promoter on the substrate surface, which adheres to the substrate surface by non-covalent bonding;
(b) applying a functional overlayer to the treated substrate surface; and
(c) curing the adhesion promoter by means of heat and/or UV radiation to form covalent bonds between the substrate surface and the adhesion promoter and between the adhesion promoter and the functional overlayer.

The method of the present invention is very straightforward and effective in providing a durable coating to a substrate surface. Depending on the functional overlayer chosen, it is possible to provide the substrate with a variety of different functional properties, such as anti-fog or anti-bacterial properties. In a preferred embodiment, the substrate surface is treated with the adhesion promoter of the present invention and subsequently rinsed with an appropriate liquid, such as water, acetone, ethanol, methanol, 2-propanol, *N,N*-dimethylformamide, dimethylsulfoxide, and mixtures thereof, for instance. Thereby, it is possible to obtain essentially a monolayer of the functionalized macromolecule on the substrate surface. This allows for an even better control of the density of the photoactive groups on the surface and for the formation of coatings with a defined thickness.

In a further aspect, the present invention also relates to a substrate which is coated with an adhesion promoter based on the functionalized macromolecule of the present invention and with a functional overlayer, wherein the adhesion promoter is covalently bound to the substrate and the functional overlayer. Again, the formation of the covalent bonds is prompted by heat and/or UV radiation.

The following examples are provided to further illustrate certain embodiments of the present invention.

### Examples

### Example 1: Preparation of polyallylamine-grafted-perfluorophenylazide (PAAm-g-PFPA) stock solutions with different grafting ratios g

For all examples, the grafting ratio g is defined as the number of repeating units of the polymer divided by the number of number of functionalized side chains.

Prior to the preparation of the stock solutions, PFPA-NHS was synthesized as described in Keana and Cai, J. Org. Chem. 1990, 55, pp. 3640-3647. All other reagents were obtained from commercial suppliers.

### a) Preparation of a PAAm-g-PFPA stock solution with a grafting ratio g of about 4:

10.0 mg (0.107 mmol) PAAm•HCl and 25 mg K₂CO₃ were dissolved in 2 ml of water. 2 ml of a 4.44 mg/ml PFPA-NHS solution in ethanol (10 mg in 2.25 ml ethanol, short time ultrasound for complete dissolution) were added and the mixture was vigorously stirred over night to yield a stock solution of 2.5 mg/ml (based on PAAm).

### b) Preparation of a PAAm-g-PFPA stock solution with a grafting ratio g of about 6:

10.0 mg (0.107 mmol) PAAm•HCl and 25 mg K₂CO₃ were dissolved in 2 ml of water. 1.33 ml of a 4.44 mg/ml PFPA-NHS solution in ethanol (10 mg in 2.25 ml ethanol) were added and the mixture was vigorously stirred over night. 0.66 ml of ethanol were added to yield a stock solution of 2.5 mg/ml (based on PAAm).

### c) Preparation of a PAAm-g-PFPA stock solution with a grafting ratio g of about 12:

10.0 mg (0.107 mmol) PAAm·HCl, 25 mg K₂CO₃ were dissolved in 2 ml of water. 0.66 ml of a 4.44 mg/ml PFPA-NHS solution in ethanol (10 mg in 2.25 ml ethanol) were added and the mixture was vigorously stirred over night. 1.33 ml of ethanol were added to yield a stock solution of 2.5 mg/ml (based on PAAm).

### Example 2: Preparation of compositions comprising PAAm-g-PFPA with different grafting ratios g

0.5 ml of one of the stock solutions described in example 1 was added to 24.5 ml of a 3:2 mixture of ethanol (14.7 ml) and buffered water (9.8 ml, HEPES (2-(4-(2-hydroxyethyl)-1-piperazinyl)-ethanesulfonic acid) 10 mM, pH 7.4).

### Example 3: Use of a composition comprising PAAm-g-PFPA as an adhesion promoter for a hydrophilic anti-fog coating on metal oxide surfaces

Several glass mirrors (10x10 cm) were washed by dipping them in a bath of water and Roche Cobas Cleaner (1:1) and subsequently rinsed with pure water. The mirror samples were ultrasonicated for 30 minutes in 2-propanol and dried with a stream of nitrogen.

The glass mirrors were suspended for one hour in the composition comprising PAAm-g-PFPA of Example 2 (g = 12). Subsequently, the mirrors were rinsed with a 3:2 mixture of ethanol and HEPES water.

A hot (80-90 °C) mixture of PVP (1300 kDa, 100 mg/ml in water) and PAAm(14)-g[4]-PFPA stock solution (Example 1) in a ratio of 1000:1 by weight was applied to the treated surface. This mixture can be sprayed, brushed, spin-coated or blasted onto the surface with a strong gas stream.

The covalent bond formation was triggered by activating the PFPA groups either by heating the sample (10 min at 170 °C), or by UV irradiation (2 min, λ = 254 nm) or both (heating followed by UV irradiation). The modified surfaces were extensively washed with pure water until complete dissolution of the non-bound hydrophilic polymer.

The water contact angle on the thus coated mirror surface was measured to be < 30°, whereas it was 54° on the non-treated mirror surface. This hydrophilic coating was resistant under a strong warm water stream for several cycles and resisted cleaning with a standard household glass cleaner. The samples stayed clear and showed no fogging even when placed directly into water vapor.

### Example 4: Use of a composition comprising PAAm-g-PFPA as an adhesion promoter for a hydrophilic anti-fog coating on silicon substrates with a natural oxide layer

Several silicon samples with a natural oxide layer (2x4 cm, 2.3 nm thick layer of SiO₂ on silicon) were cleaned in an ultrasonic bath of toluene (20 min) and subsequently 2-propanol (20 min) and then dried in a nitrogen stream. The surfaces were then plasma treated in an oxygen atmosphere for 2 minutes.

The samples were suspended in the composition comprising of PAAM-g-PFPA (example 2, g = 12) for one hour and then rinsed extensively with a 3:2 mixture of ethanol and buffered water.

A hot (80-90 °C) mixture of PVP (1300 kDa, 100 mg/ml in water) and PAAm(14)-g[4]-PFPA stock solution (Example 1) in a ratio of 1000:1 by weight was spin-coated onto the treated surface.

Activation by UV irradiation (2 min, λ = 254 nm) was performed only on part of each sample, by covering half of the substrate with a black filter. The modified surfaces were then extensively rinsed with pure water until complete removal of the non-bound polymer chains.

The layer thickness was measured after each of the above steps, using a variable angle spectroscopic ellipsometer (M-2000F EC400, J.A. Wollam Inc., Lincoln, USA) and the data was evaluated using the software WVASE32 (WexTech Systems, Inc., New York).

Static water contact angle measurements were also performed, using a Krüss contact angle measuring system (G2/G40 2.05-D, Krüss GmbH, Germany). Analyses were carried out by means of the tangent method 2 routine of the Krüss Drop-Shape Analysis program (DSA version 1.80.0.2 for Windows 9x/NT4/2000, 1997-2002 KRUESS). The results are shown in table 1.

**Table 1: Layer thickness and static water contact angles measured after the different treatment steps.**

| **Step** | **Average Thickness** | **Contact Angle** |
|---|---|---|
| a) SiO₂ substrate | 2.3 nm | 14 ± 0.5° |
| b) PAAm-g[4]-PFPA | 1.1 ± 0.2 nm | 65.9 ± 0.3° |
| c) PVP coated part | 20.1 ± 4.0 nm | 26.8 ± 0.5° |
| d) Non coated side | 1.4 ± 0.6 nm | 38.9 ± 0.2° |

In addition, the above coatings were highly resistant against nonspecific protein adsorption, which was measured by ellipsometry. The increase in layer thickness after incubation in full human serum was less than 0.1 nm, which is below the detection limit of ellipsometry. Tribological tests on these surfaces using a PDMS countersurface showed an extremely low coefficient of friction of 0.04 ± 0.02.

### Example 5: Use of a composition comprising PAAm-g-PFPA for crosslinking a hydrogel covalently to a substrate

Glass samples (10x10 cm) were washed in a bath of water and Cobas Cleaner (1:1) and rinsed with pure water. The samples were ultrasonicated for 30 minutes in isopropanol and dried with a stream of nitrogen.

Subsequently, the samples were suspended for one hour in the composition of example 2. The surfaces were rinsed with a 3:2 mixture of ethanol and buffered water.

A hot (80-90 °C) mixture of PVP (1300 kDa, 100mg/ml in water) and PAAm-g[4]-PFPA stock solution (example 1) in a ratio of 100:1 by weight was applied to the treated surface. This solution can be sprayed, brushed, spin-coated or blasted over the surface with a strong gas stream.

The covalent attachment and cross-linking was triggered by activating the PFPA by UV irradiation (2 min, λ = 254 nm). The modified surfaces were extensively washed with pure water until complete dissolution of the non-bound hydrophilic polymer.

The water contact angle on the treated surface was measured to be below 30° whereas it was > 50°on the non-treated part of oxide surface. This hydrophilic coating had a hydrogel conformation with a very low coefficient of friction (-0.1) and resisted washing with boiling water for more than 60 minutes.

### Example 6: Use of a composition comprising PAAm-g[4]-PFPA as an adhesion promoter for anti-fog coating of polycarbonates

Polycarbonate samples (10x10 cm) were washed in a bath of water and Cobas Cleaner (1:1) and rinsed with pure water. The samples were sonicated for 30 minutes in isopropanol and dried with a stream of nitrogen.

Subsequently, the samples were suspended for one hour in the composition comprising PAAm-g[4]-PFPA of example 2. The surfaces were rinsed with a 3:2 mixture of ethanol and buffered water.

A solution of PVP (1300 kDa, 100mg/ml in water) and PAAm-g[4]-PFFA stock solution (example 1) in a ratio of 100:1 by weight was applied to the treated surface at 80-90 °C. This solution can be sprayed, brushed, spin-coated or blasted over the surface with a strong gas stream.

The covalent attachment and cross-linking was triggered by activating the PFPA either by heating the sample (10 min at 170 °C), by UV irradiation (2 min, λ = 254 nm) or both (heating followed by UV irradiation). The modified surfaces were extensively washed with pure water until complete dissolution of the non-bound hydrophilic polymer. The water contact angle on the treated surface was measured to be below 30° whereas it was > 78°on the non-treated polycarbonate surface. This hydrophilic coating had a hydrogel conformation with a very low coefficient of friction (∼0.1) and resisted washing with boiling water for more than 60 minutes. The samples stayed clear and showed no fogging even when placed directly into water vapor.

### Example 7 (not part of the claimed inventive) : Preparation of a bovine-serum-albumin-grafted-perfluorophenylazide (BSA-g-PFPA) stock solution

Prior to the preparation of the stock solution, PFPA-NHS was synthesized as described in Keana and Cai, J. Org. Chem. 1990, 55, pp. 3640-3647. All other reagents were obtained from commercial suppliers.

BSA (25 mg, 0.0003 mmol) and K₂CO₃ (62.5 mg, 0.45 mmol) were dissolved in pure water (5 ml, Milli-Q Advantage 10). After complete dissolution of BSA, a solution of PFPA-NHS (5.85 mg, 0.0167 mmol) in ethanol (5 ml) was added to the BSA solution. The mixture was let to react overnight at room temperature under vigorous stirring. Each BSA was functionalized in average with about 42 PFPA photoactive units.

### Example 8: (not part of the claimed invention) Preparation of compositions comprising BSA-g-PFPA

0.5 ml of the stock solution described in example 7 was added to 14.5 ml of buffered water (HEPES 10 mM, pH 7.4).

### Example 9: (not part of the claimed invention) Use of a composition comprising BSA-g-PFPA as an adhesion promoter for a hydrophilic anti-fog coating on metal oxide surfaces

Several glass mirror samples (10x10 cm) were washed by dipping them in a bath of water and Roche Cobas Cleaner (1:1) and subsequently rinsed with pure water. The mirror samples were ultrasonicated for 30 minutes in 2-propanol and dried with a stream of nitrogen.

The glass mirrors were suspended for one hour in the composition comprising BSA-g-PFPA of example 8. Subsequently, the samples were dried under nitrogen.

A solution of PVP (1300 kDa, 100 mg/ml in water) and BSA-g-PFPA stock solution (Example 7) in a ratio of 1000:1 by weight was applied to the treated surface at 80-90 °C. This solution can be sprayed, brushed, spin-coated or blasted onto the surface with a strong gas stream.

The covalent bond formation was triggered by activating the PFPA groups either by heating the sample (10 min at 170 °C), or by UV irradiation (2 min, λ = 254 nm) or both (heating followed by UV irradiation). The modified surfaces were extensively washed with pure water until complete dissolution of the non-bound hydrophilic polymer.

The water contact angle on the thus coated mirror surface was measured to be < 30°, whereas it was 54° on the non-treated mirror surface. This hydrophilic coating was resistant under a strong cold water stream for 50 cycles. The samples stayed clear and showed no fogging even when placed directly into water vapor.

### Example 10: (not part of the claimed invention) Use of a composition comprising BSA-g-PFPA as an adhesion promoter for a nail enhancement

BSA (25 mg, 0.0003 mmol) and K₂CO₃ (62.5 mg, 0.45 mmol) were dissolved in pure water (5 ml, Milli-Q Advantage 10). After complete dissolution of BSA, a solution of PFPA-NHS (5.85 mg, 0.0167 mmol) in ethanol (5 ml) was added to the BSA solution. The mixture was let react overnight at room temperature under vigorous stirring.

Each BSA was thus functionalized in average by 42 PFPA photolinkers. The resulting solution of BSA-g-PFPA was diluted with acetone (5 ml) and ethanol (230 ml). This solution was stored in black glass bottles and used as is for further application.

The above solution was brushed on fingernails in a specialized nail studio. After a short air-drying, the artificial nail consisting of a UV curable polymer mixture was applied to the treated nail, modeled, and cured (λ = 360 nm) according manufacturer's instructions for the artificial nail.

The adhesion of the modeling gels on BSA-PFPA coated nails was for the large majority of the tested nails (data for approximately 30 individuals) maintained for a period of more than 28 days. In the absence of the coating, one third of the tested modeled gel coatings was lost within 20 days from application.

### Example 11: (not part of the claimed invention) Use of a composition comprising BSA-g-PFPA as a fingernail hardener

Keratin (5 mg/ml) was added to the BSA-g-PFPA/acetone/ethanol solution of example 10 and mixed until fully dissolved.

This mixture was brushed on fingernails. After short air-drying, the nails are irradiated with UV-light (λ = 360 nm) for 5 minutes. The treated nails showed a strong reluctance towards braking and cracking compared to untreated nails for a period of more than 7 days.

### Example 12: (not part of the claimed invention) Use of a composition comprising BSA-g-PFPA as an adhesion promoter for coating Teflon® with PEG

Teflon samples (10x10 cm) were washed in a bath of water and Cobas Cleaner (1:1) and rinsed with pure water. The samples were suspended for one hour in the BSA-PFPA composition of example 8. The surfaces were then dried with a stream of nitrogen.

A solution of PEG (1000 kDa, 25 mg/ml in water) was applied to the treated surface. This solution can be sprayed, brushed, spin-coated or blasted over the surface with a strong gas stream.

The covalent attachment was triggered by activating the PFPA either by heating the sample (10 min at 170 °C), or by UV irradiation (2 min, λ = 254 nm) or both (heating followed by UV irradiation). The modified surfaces were extensively washed with pure water until complete dissolution of the non-bound hydrophilic polymer.

The water contact angle on the treated surface was measured to be about 30°, whereas it was 110°on the non-treated Teflon. This hydrophilic coating was resisting more than 10 washing cycles in a chemical washing machine.

### Example 13: (not part of the claimed invention) Use of a composition comprising BSA-g-PFPA as an adhesion promoter for coating Teflon® with PVP

Teflon samples (10x10 cm) were washed in a bath of water and Cobas Cleaner (1:1) and rinsed with pure water. The samples were suspended for one hour in the BSA-PFPA composition of example 8. The surfaces were then dried with a stream of nitrogen.

A solution of PVP (1300 kDa, 100 mg/ml in water) was applied to the treated surface. This solution can be sprayed, brushed, spin-coated or blasted over the surface with a strong gas stream.

The covalent attachment was triggered by activating the PFPA either by heating the sample (10 min at 170 °C), or by UV irradiation (2 min, λ = 254 nm) or both (heating followed by UV irradiation). The modified surfaces were extensively washed with pure water until complete dissolution of the non-bound hydrophilic polymer.

The water contact angle on the treated surface was measured to be about 34°, whereas it was 122°on the non-treated Teflon. This hydrophilic coating was resisting more than 10 washing cycles in a chemical washing machine.

### Example 14: (not part of the claimed invention) Use of a composition comprising BSA-g-PFPA as an adhesion promoter for coating polypropylene with PVP

Polypropylene samples (10x10 cm) were washed in a bath of water and Cobas Cleaner (1:1) and rinsed with pure water. The samples were suspended for 1 hour in the BSA-PFPA composition of example 8. The surfaces were then dried with a stream of nitrogen.

A solution of PVP (1300 kDa, 100 mg/ml in water) was applied to the treated surface. This solution can be sprayed, brushed, spin-coated or blasted over the surface with a strong gas stream.

The covalent attachment was triggered by activating the PFPA either by heating the sample (10 min at 170 °C), or by UV irradiation (2 min, λ = 254 nm) or both (heating followed by UV irradiation). The modified surfaces were extensively washed with pure water until complete dissolution of the non-bound hydrophilic polymer.

The water contact angle on the treated surface was measured to be about 32°, whereas it was 110°on the non-treated polypropylene. This hydrophilic coating was resisting more than 10 washing cycles in a chemical washing machine.

### Example 15: (not part of the claimed invention) Use of BSA-g-PFPA as an adhesion promoter for an anti-fog coating on polycarbonate

Polycarbonate samples (10x10 cm) were washed in a bath of water and Cobas Cleaner (1:1) and rinsed with pure water. They were sonicated for 30 min in 2-propanol and dried with nitrogen.

The samples were suspended for 1 hour in the BSA-PFPA composition of example 8. The surfaces were then dried with a stream of nitrogen.

A mixture of PVP (1300 kDa, 100 mg/ml in water) and the BSA-PFPA stock solution (example 6) in a ratio of 1000:1 by weight was applied to the treated surface at 80-90 °C. This solution can be sprayed, brushed, spin-coated or blasted over the surface with a strong gas stream.

The covalent attachment was triggered by activating the PFPA by UV irradiation (2 min, λ = 254 nm). The modified surfaces were extensively washed with pure water until complete dissolution of the non-bound hydrophilic polymer.

The water contact angle on the treated surface was measured to be below 30°, whereas it was 78°on the non-treated polycarbonate. This hydrophilic coating was resistant under strong cold water stream for 50 cycles.

### Example 16: Modification of well plates with dextran (2 000 kDa)

Culture polystyrene (PS) well plates (24 wells) activated by oxygen plasma (2 min) were modified with a composition comprising PAAm(14)-g[4]-PFPA (1 ml).

An aqueous solution of dextran 2 000 kDa (50 µL, 100 mg/ml, Dextran T2000 from Pharmacos) was introduced into the wells and dried with a strong nitrogen stream until complete dryness. Immobilization of the dextran was performed by UV irradiation (2 min, λ = 254 nm), and subsequently, the plates were washed extensively with pure water.

The wells were conditioned by filling them with HEPES buffer (2 ml) for 24 hours at 4 °C, washed with pure water and dried. X-ray photoelectron spectroscopy measurements (XPS) showed effective grafting of dextran onto the surface.

### Example 17: Modification of well plates with PVP (1 300 kDa)

Culture polystyrene (PS) well plates (24 wells) activated by oxygen plasma (2 min) were modified with a composition comprising PAAm(14)-g[4]-PFPA (1, ml).

An aqueous solution of PVP 1 300 kDa (50 µL, 100 mg/ml, PVP 1300 KDa from Aldrich) was introduced into the wells and dried with a strong nitrogen stream until complete dryness. Immobilization of the PVP was performed by UV irradiation (2 min, λ = 254 nm), and subsequently, the plates were washed extensively with pure water.

The wells were conditioned by filling them with HEPES buffer (2 ml) for 24 hours at 4 °C, washed with pure water and dried. X-ray photoelectron spectroscopy measurements (XPS) showed effective grafting of PVP onto the surface.

### Example 18: Modification of well plates with polyethylene glycol (35 kDa)

Culture polystyrene (PS) well plates (24 wells) activated by oxygen plasma (2 min) were modified with a composition comprising PAAm(14)-g[4]-PFPA (1 ml).

An aqueous solution of PEG 35 kDa (50 µL, 500 mg/ml, PEG 35 kDa from Fluka) was introduced into the wells and dried with a strong nitrogen stream until complete dryness. Immobilization of the PVP was performed by UV irradiation (2 min, A = 254 nm), and subsequently, the plates were washed extensively with pure water.

The wells were conditioned by filling them with HEPES buffer (2 ml) for 24 hours at 4 °C, washed with pure water and dried. X-ray photoelectron spectroscopy measurements (XPS) showed effective grafting of PEG onto the surface.

### Example 19: Cell tests with modified well plates

In well plates modified as described in examples 16, 17, and 18, respectively, 3T3 fibroblast cells (10³ cells/ml; 500 µl/24 well) were incubated for different time periods (up to 28 days).

In a control experiment, unmodified wells and PAAm(14)-g[4]-PFPA treated wells (UV activation without polymer on it) were examined.

Dextran and PVP modified well plates stayed resistant towards cell attachment for the full timescale of the experiment (more than 3 weeks), whereas PEG 35 was not fully resistant, probably due to the lower molecular weight.

Both control experiments with and without coating of the wells with PAAm(14)-g[4]-PFPA showed extensive and almost equal cell attachment and spreading. Live/dead staining showed also no difference between the two control substrates, confirming the non-cell toxicity of the coating.

### Example 20: Modification of a Teflon® membrane with silicone

A series of Teflon samples has been prepared and studied by XPS in order to show that it is possible to covalently link a polydimethylsiloxane polymer (PDMS) onto Teflon, thanks to the photoactive adhesion promoter composition prepared in example 2 with g = 4.

For the experiments, a fluoropolymer net (Tetex) from Franz Eckert GmbH was used.

The following samples have been prepared and analyzed:

| | |
|---|---|
| Sample 1: | Tetex as received |
| Sample 2: | Tetex after 2 minutes 02 plasma |
| | (plasma) |
| Sample 3: | Tetex, plasma, PAAm-g-PFPA |
| Sample 4: | Telex, plasma, PAAm-g-PFPA, PDMS, UV and extracted with ethyl acetate |
| Sample 5: (control 1) | same as sample 4, but no PAAm-g-PFPA |
| Sample 6: (control 2) | same as sample 4, but no plasma and nc PAAm-g-PFPA |

The adsorption of PAAm-g-PFPA (samples 3, 4, 5) was performed by immersing the foil for 30 minutes in a composition according to example 2. Then, the composition was exchanged three times with a 3:2 mixture of ethanol and HEPES buffered water, before the samples were rinsed with milli-Q water and blown dry.

For modification with PDMS, the samples were soaked in SYLGARD184 base without curing agent, subjected to UV irradiation (254 nm) for 5 minutes, and afterwards extensively extracted with warm ethyl acetate and 2-propanol to remove none-bound PDMS.

The covalently bound PDMS was easily detected by XPS on sample 4. On sample 5 and 6 without PAAm-g-PFPA, only traces of silicon were detected (Table 2). Nitrogen Carbon Fluorine Oxygen Silicon

**Table 2: normalized atomic concentration measured by XPS (only elements from the top 5-10 nm of the samples are detected).**

| | Nitrogen | Carbon | Fluorine | Oxygon | Silicon |
|---|---|---|---|---|---|
| sample 1 | 0.7 | 66.8 | 28.5 | 3.1 | 0.8 |
| sample 2 | 0.2 | 56.8 | 32.7 | 9.9 | 0.3 |
| sample 3 | 5.3 | 63.8 | 21.2 | 9.4 | 0.2 |
| sample 4 | 3.6 | 54.7 | 12.2 | 18.1 | 11.4 |
| sample 5 | 0.4 | 56.9 | 32.4 | 9.5 | 0.8 |
| sample 6 | 0.1 | 56.8 | 41.0 | 1.8 | 0.3 |

### Example 21: (not part of the claimed invention) Printing on Teflon®

Teflon samples (10x10 cm) were washed in a bath of water bath and Cobas Cleaner (1:1) and rinsed with pure water.

One half of each sample was suspended for 1 hour in the BSA-PFPA composition of example 8. The surfaces were then dried with a stream of nitrogen.

A motif was printed on both the coated and uncoated halfs using pad printing.

The covalent attachment was triggered by activating the PFPA either by heating the sample (10 min at 170 °C), or by UV light (5 min, λ = 254 nm) or both (heating followed by UV light).

Treatment of the samples with a solvent (cyclohexanone) revealed a much stronger attachment of the printing to the BSA-PFPA surface compared to the non-treated areas.

### Example 22: (not part of the claimed invention) Printing on polyethyleneimine modified Teflon®

Teflon samples (10x10 cm) were washed in a bath of water bath and Cobas Cleaner (1:1) and rinsed with pure water.

One half of each sample was suspended for 1 hour in the BSA-PFPA composition of example 8. The surfaces were then dried with a stream of nitrogen.

A solution of polyethyleneimine (25 kDa) in chloroform (100 mg/ml) was applied on top.

The covalent attachment was triggered by activating the PFPA either by heating the sample (10 min at 170 °C), or by UV light (5 min, λ = 254 nm) or both (heating followed by UV light). The modified surfaces were extensively rinsed with pure water until complete dissolution of the non-bound polyethyleneimine. The samples thus obtained comprised a PEI coating only on the one half that had been treated with the BSA-PFPA composition.

A motif was printed on both the coated and uncoated halfs using pad printing.

Adhesion tests of the print color on the coated and uncoated halfs using a scotch tape showed a much stronger adhesion of the color to the coated part of the samples.

## Claims

1. A functionalized macromolecule comprising a polymeric core and at least two functionalized side chains,
**characterized in that**
the polymeric core comprises a synthetic polymer selected from the group consisting of polyallylamine and polyethyleneimine,
the at least two functionalized side chains are linked to the polymeric core by a linker group A selected from the group consisting of a secondary or tertiary amine, an ether, a thioether, a carboxylic acid ester, an amide, and a thioester, and that
the at least two functionalized side chains comprise a photoreactive group,
wherein said photoreactive group is

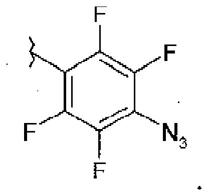

2. Functionalized macromolecule according to claim 1, **characterized in that** the linker group A is an amide.

3. Functionalized macromolecule according to one of claims 1 to 2, **characterized in that** the polymeric core comprises on average a functionalized side chain on at least every 24^{th}, preferably on at least every 12^{th}, more preferably on at least every 4^{th} repeating unit or amino acid unit, respectively.

4. Composition comprising the functionalized macromolecule of one of claims 1 to 3 and a liquid, preferably selected from the group consisting of water, acetone, ethanol, methanol, 2-propanol, *N,N-*dimethylformamide, dimethylsulfoxide, and mixtures thereof.

5. Composition according to claim 4, further comprising an additive selected from the group consisting of polyethylene glycol, polyvinylpyrrolidone, dextran, fatty acids, keratin, collagen, and mixtures thereof.

6. Use of the composition according to claim 4 or 5 as an adhesion promoter.

7. Use of the composition according to claim 4 or 5 as an adhesion promoter for nail enhancements.

8. Use of the composition according to claim 4 or 5 as a fingernail hardener.

9. Use of the composition according to claim 4 or 5 as an adhesion promoter for hydrophobic polymer substrates.

10. Use of the composition according to claim 4 or 5 as an adhesion promoter for an anti-fog coating.

11. Method for applying a functional coating to a substrate surface, comprising the steps of:
(a) treating the substrate surface with an adhesion promoter comprising the functionalized macromolecule of one of claims 1 to 3 to form a film of the adhesion promoter on the substrate surface, which adheres to the substrate surface by non-covalent bonding;
(b) applying a functional overlayer to the treated substrate surface; and
(c) curing the adhesion promoter by means of heat and/or UV radiation to form covalent bonds between the substrate surface and the adhesion promoter and between the adhesion promoter and the functional overlayer.

12. A substrate which is coated with an adhesion promoter based on the functionalized macromolecule of one of claims 1 to 3 and with a functional overlayer, wherein the adhesion promoter is covalently bound to the substrate and the functional overlayer.

## Patentansprüche

1. Ein funktionalisiertes Makromolekül enthaltend einen polymeren Kern und wenigstens zwei funktionelle Seitenketten, **dadurch gekennzeichnet dass**
der polymere Kern ein synthetisches Polymer enthält, das ausgewählt ist aus der Gruppe bestehend aus Polyallylamin und Polyethylenimin,
wobei die wenigstens zwei funktionalisierten Seitenketten mit dem polymeren Kern durch eine Verbindungsgruppe A verbunden ist, die ausgewählt ist bestehend aus einem sekundären oder einem tertiären Amin, einem Ether, einem Thioether, einem Carbonsäureester, einem Amid und einem Thioester, und dass
die wenigstens zwei funktionalisierten Seitenketten eine photoreaktive Gruppe enthalten,
wobei diese photoreaktive Gruppe 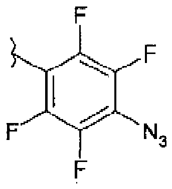 ist.

2. Funktionalisiertes Makromolekül nach Anspruch 1, **dadurch gekennzeichnet, dass** die Verbindungsgruppe A ein Amid ist.

3. Funktionalisiertes Makromolekül nach einem der Ansprüche 1 bis 2, **dadurch gekennzeichnet, dass** der polymere Kern im Durchschnitt eine funktionalisierte Seitenkette bei wenigstens jeder 24-igsten, vorzugsweise jeder 12-ten, besonders bevorzugt bei jeder 4-ten Wiederholungseinheit beziehungsweise Aminosäureeinheit aufweist.

4. Zusammensetzung, enthaltend das funktionalisierte Makromolekül nach einem der Ansprüche 1 bis 3 und eine Flüssigkeit, vorzugsweise ausgewählt aus der Gruppe bestehend aus Wasser, Aceton, Ethanol, Methanol, 2-Propanol, N,N-Dimethylformamid, Dimethylsulfoxid und Mischungen davon.

5. Zusammensetzung nach Anspruch 4, weiter enthaltend einen Zusatz ausgewählt aus der Gruppe bestehend aus Polyethylenglykol, Polyvinylpyrrolidon, Dextran, Fettsäure, Keratin, Kollagen und Mischungen davon.

6. Verwendung der Zusammensetzung nach Anspruch 4 oder 5 als Haftvermittler.

7. Verwendung der Zusammensetzung nach Anspruch 4 oder 5 als Haftvermittler für Nagelerweiterungen.

8. Verwendung der Zusammensetzung nach Anspruch 4 oder 5 als Fingernagel-Härter.

9. Verwendung der Zusammensetzung nach Anspruch 4 oder 5 als Haftvermittler für hydrophobe Polymersubstrate.

10. Verwendung der Zusammensetzung nach Anspruch 4 oder 5 als Haftvermittler für eine Antihaft-Beschichtung.

11. Verfahren zum Aufbringen einer funktionellen Beschichtung auf eine Substratoberfläche, enthaltend die schritte von:
(a) Behandlung der Substratoberfläche mit einem Haftvermittler enthaltend die funktionellen Makromoleküle gemäss einem der Ansprüche 1 bis 3 zur Bildung eines Films des Haftvermittlers auf der Substratoberfläche, welche durch nichtkovalente Bindung an der Substratoberfläche haftet;
(b) Aufbringen einer funktionellen Deckschicht auf die behandelte Oberfläche; und
(c) Härtung des Haftvermittlers durch Hitze und/oder UV-Strahlung zur Bildung von kovalenten Bindungen zwischen der Substratoberfläche und des Haftvermittlers und zwischen dem Haftvermittler und der funktionellen Deckschicht.

12. Ein Substrat, das mit einem Haftvermittler, welcher auf dem funktionalisierten Makromolekül gemäss einem der Ansprüche 1 bis 3 basiert, und mit einer funktionellen Deckschicht beschichtet ist, wobei der Haftvermittler kovalent an das Substrat und die funktionalle Deckschicht gebunden ist.

## Revendications

1. Une macromolécule fonctionnalisée comprenant un noyau polymérique et au moins deux chaînes latérales fonctionnalisées,
**caractérisée en ce que**
le noyau polymérique comprend un polymère synthétique sélectionné dans le groupe consistant en polyallylamine et polyéthylènimine,
les au moins deux chaînes latérales fonctionnalisées sont reliées au noyau polymérique par un groupe de liaison A sélectionné dans le groupe consistant en amine secondaire ou tertiaire, éther, thioéther, ester d'acide carboxylique, amide, et thioester, et **en ce que**
les au moins deux chaînes latérales fonctionnalisées comprennent un groupe photoréactif,
ledit groupe photoréactif étant

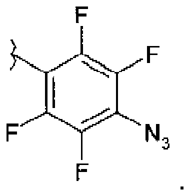

2. Macromolécule fonctionnalisée selon la revendication 1, **caractérisée en ce que** le groupe de liaison A est un amide.

3. Macromolécule fonctionnalisée selon l'une des revendications 1, à 2, **caractérisée en ce que** le noyau polymérique comprend en moyenne une chaîne latérale fonctionnalisée sur au moins chaque 24^{éme}, préférablement sur au moins chaque 12^{éme}, de façon plus préférée sur au moins chaque 4^{éme} unité récurrente ou unité d'acides aminés, respectivement.

4. Composition comprenant la macromolécule fonctionnalisée selon l'une des revendications 1 à 3 et un liquide, préférablement sélectionné dans le groupe consistant en eau, acétone, éthanol, méthanol, 2-propanol, *N,N*-diméthylformamide, diméthylsulfoxide, et des mélanges de ceux-ci.

5. Composition selon la revendication 4, comprenant en outre un additif sélectionné dans le groupe consistant en polyéthylèneglycol, polyvinylpyrrolidone, dextrane, acides gras, kératine, collagène, et des mélanges de ceux-ci.

6. Utilisation de la composition selon les revendications 4 ou 5 comme promoteur d'adhésion.

7. Utilisation de la composition selon les revendications 4 ou 5 comme promoteur d'adhésion pour la pose d'ongles.

8. Utilisation de la composition selon les revendications 4 ou 5 comme un durcisseur d'ongles.

9. Utilisation de la composition selon les revendications 4 ou 5 comme promoteur d'adhésion pour des substrats de polymère hydrophobe.

10. Utilisation de la composition selon les revendications 4 ou 5 comme promoteur d'adhésion pour un revêtement antibuée.

11. Méthode pour appliquer un revêtement fonctionnel à une surface d'un substrat, comprenant les étapes:
(a) traiter la surface du substrat avec un promoteur d'adhésion comprenant la macromolécule fonctionnalisée selon l'une des revendications 1 à 3 pour former un film de promoteur d'adhésion à la surface du substrat qui adhère à la surface du substrat par des liaisons non-covalentes;
(b) appliquer une surcouche fonctionnelle à la surface traitée du substrat; et
(c) durcir le promoteur d'adhésion au moyen de chaleur et/ou de radiations UV pour former des liaisons covalentes entre la surface du substrat et le promoteur d'adhésion et entre le promoteur d'adhésion et la surcouche fonctionnelle.

12. Un substrat qui est recouvert d'un promoteur d'adhésion basé sur la macromolécule fonctionnalisée selon l'une des revendications 1 à 3 et avec une surcouche fonctionnelle, le promoteur d'adhésion étant lié de façon covalente au substrat et à la surcouche fonctionnelle.
